# EUROPEAN PATENT APPLICATION

(11) **EP 1 905 445 A1**
(43) Date of publication of application: **02.04.2008**
(21) Application number: 06757068.9
(22) Date of filing: 06.06.2006
(51) Int. Cl.: A61K 38/00, A61P 1/16, A61P 35/00

(54) **TUMOR GROWTH INHIBITOR**

(30) Priority: 06.06.2005 JP 2005165295
(71) Applicant: Protein Express Co., Ltd., Choshi-shi, Chiba 288-0041 (JP)
(72) Inventor: ISHII, Masaru, Saitama 3370051 (JP); KIYONO, Yuko, Kita-ku Saitama-shi, Saitama 3310813 (JP); YOKOTA, Harumasa, 0510004 (JP); ICHIHARA, Atsuhiro, Kanagawa 2160033 (JP); ISHIDA, Yuichi, Washimiya-machi, Kitakatsushika-gun, Sai (JP)
(74) Representative: Krauss, Jan
(86) International application number: PCT/JP2006/311329
(87) International publication number: WO 2006/132245

(57) **Abstract**

The subject of the present invention is to provide a novel tumor-growth suppressor. Such a subject is solved by a tumor-growth suppressor comprising as an active ingredient a polypeptide consisting of a partial sequence selected within a sequence from the 1st to the 18th in the N-terminus of amino acid sequence in the prorenin profragment region or equivalent peptide thereof. The partial sequence is preferably a sequence of 2 to 8 amino acids. It is believed that the tumor-growth suppressor of the present invention can show higher tumor-growth suppressive effects than conventional angiotensin-converting enzyme inhibitors and the like.

## Description

### Technical Field

The present invention relates to a tumor-growth suppressor comprising as an active ingredient a polypeptide consisting of a partial sequence selected within a sequence from the 1st to the 18th in the N-terminus of an amino acid sequence in the prorenin profragment region or equivalent peptide thereof.

The present application claims the priority of Japanese Patent Application No. 2005-165295, which is incorporated herein by reference.

### Background Art

Tumor-growth suppressors are classified into several categories such as alkylating agents, antibiotics capable of affecting nucleic acids (doxorubicin etc.), platinum complexes, alkaloid antitumor agents, antimetabolites (5-fluorouracil etc.), topoisomerase inhibitors, biological reaction-adjusting agents (interferon etc.) and hormone therapeutic agents (tamoxifen etc.). These anticancer drugs show growth suppressive effects directly on tumor cells through various kinds of mechanism of action.

Recently, it is becoming obvious that endogenous angiotensin II (Ang II) is involved in tumor angiogenesis. While Ang II-producing enzyme inhibitor and Ang II receptor antagonist respectively have almost equivalent effects on organ protection, it has been pointed out that they may also have tumor-growth suppressive effects. Angiotensin-converting enzyme inhibitor (ACE inhibitor) has inhibiting effects on ACE which is an enzyme which converts angiotensin I (Ang I) to Ang II, and it was reported that patients continuously taking ACE inhibitor had a significantly low death rate from cancers. Further, it was confirmed by using Ang II receptor antagonist and Ang II receptor knockout mice that Ang II is involved in angiogenesis and tumor growth (Non-Patent Documents No. 1 and 2).

There are circulating RAS and tissue RAS in Renin-angiotensin system (RAS) producing Ang II. Independently of physiologically circulating RAS which is essential for maintaining homeostasis such as blood pressure and body fluid of a living body, it is believed that the activation of RAS in tissues in a clinical state increases the concentration of Ang II in the tissues and is deeply involved in the onset of and progress of hypertension, diabetes complication, renal disease, cardiac failure and etc. by the fact that the increased Ang II specifically works through its receptor. Conventionally, ACE inhibitor and Ang II receptor antagonist have been employed as a hypotensor, but owing to the re-increase of Ang II or the Ang II effect, which is so-called "escape phenomenon," it has been pointed out that therapeutic effects on their single use have limitations.

Renin is a rate-limiting enzyme which produces Ang I in RAS. Renin is biosynthesized mainly in kidney as pre-pro-renin, which is the precursor of renin, consisting of 406 amino acids. Then, 23 amino acids in N-terminus are cleaved to produce prorenin. Then, a fragment (referred to as "profragment" or "pf") of 43 amino acids is further cleaved from N-terminus, generating renin (also referred to as "mature renin") consisting of 340 amino acids. Usually, prorenin exhibits no enzyme activity, but it has been known that on binding in vitro to an antibody which specifically recognizes the profragment site described above to form an immune complex, prorenin starts to express an enzyme activity (renin-like activity) (Patent Documents No. 1 and 2).

The present inventors previously found that the activation of tissue RAS can be controlled by administering a partial peptide derived from prorenin profragment to disease animal models of streptozotocin (STZ)-induced diabetes and also found that the progress of the sclerosis lesion of renal glomeruli caused by diabetes can be suppressed by administering a partial peptide derived from prorenin profragment to disease animal models of STZ-induced diabetes (Patent Document No. 3).
However, there are no reports on a tumor-growth suppressor containing as an active ingredient a partial peptide derived from prorenin profragment.
(Patent Document No. 1) Japanese Patent No. 3271157
(Patent Document No. 2) U.S. Patent No. 5945512
(Patent Document No. 3) International Publication WO2004/073740
(Non-Patent Document No. 1) "Igaku no Ayumi (Footstep of Medicine)," 207: 35-40, (2003)
(Non-Patent Document No. 2) Biochem. Biophys. Res. Commun, 294: 441-447, (2002)

### Disclosure of the Invention

### [Problems to be solved]

The subject of the present invention is to provide a novel tumor-growth suppressor.

### (Means to solve the problems)

The present inventors have found that a polypeptide consisting of a partial sequence selected within a sequence from the 1st to the 18th in the N-terminus of an amino acid sequence in the prorenin profragment region has tumor-growth suppressive effects, thereby making it possible to provide a novel tumor-growth suppressor.

Therefore, the present invention consists of the following inventions:
1. A tumor-growth suppressor comprising as an active ingredient a polypeptide consisting of a partial sequence selected within a sequence from the 1st to the 18th in the N-terminus of amino acid sequence in the prorenin profragment region or equivalent peptide thereof.
2. The tumor-growth suppressor according to the preceding aspect 1, wherein the partial sequence is a sequence of 2 to 8 amino acids.
3. The tumor-growth suppressor according to the preceding aspects 1 or 2, wherein the partial sequence is an amino acid sequence comprising at least any one of the sequences from the 11th to the 13th, from the 13th to the 15th and from the 11th to the 15th in the N-terminus.
4. The tumor-growth suppressor according to any one of the preceding aspects 1 to 3, wherein the partial sequence is an amino acid sequence from the 11th to the 18th in the N-terminus.
5. The tumor-growth suppressor according to any one of the preceding aspects 1 to 4, wherein the tumor-growth suppressor is used for a solid tumor.
6. The tumor-growth suppressor according to the preceding aspect 5, wherein the tumor-growth suppressor is used for liver cancer.

### (Effects of the Invention)

The novel tumor-growth suppressor of the present invention can provide more excellent tumor-growth suppressive effects than conventionally known ACE inhibitors and the like. Usually, the present tumor-growth suppressor used for treating tumor is more likely to be highly effective for sick patients showing no or few effects through the use of other tumor-growth suppressors comprising ACE inhibitor and the like.

### Brief Description of the Drawings

Figure 1 shows the comparison among final body weights of mice.
Figure 2 shows chronological volume changes (mm³) in the average tumor volumes of human liver cancer-implanted nude mice.

### (Explanation of Symbols)

- a: saline
- b: captopril 10 mg/kg
- c: pf11-18 peptide 1 mg/kg
- d: pf11-18 peptide 10 mg/kg

### Description of the Preferred Embodiment

The present invention will be explained in detail below, and the technical and scientific terms used herein have, unless otherwise specified, the meanings as commonly understood by those ordinarily skilled in the art to which this invention pertains. Various methods known to those skilled in the art are mentioned herein for reference. Documents such as publications disclosing those well known methods cited are incorporated herein by reference to the extent that each publication was denoted in their entirety.

The present invention relates to a tumor-growth suppressor comprising as an active ingredient a polypeptide consisting of a partial sequence selected within a sequence from the 1st to the 18th in the N-terminus of an amino acid sequence in the prorenin profragment site or equivalent peptide thereof.

Antihuman pf antibody, which is an antibody specifically recognizing a human prorenin profragment site, activates human prorenin in vitro. A human pf peptide derived from the human prorenin profragment site is known to antagonistically inhibit the in vitro activation of human prorenin induced by the antihuman pf antibody. In the same manner, a rat pf peptide, which is the antigen of antirat pf antibody, can antagonistically inhibit the in vitro activation of rat prorenin induced by the antirat pf antibody. Thus, these peptides can inhibit the activation of rat prorenin, wherein change in primary structure caused by interactions between proteins is not induced but conformational change induced (sometimes referred to as "nonenzymatic activation of prorenin"), by antagonistically inhibiting the binding of the prorenin and a prorenin-binding protein.

The present inventors previously reported that the administration of a partial peptide derived from a rat prorenin profragment to hypertensive rat (SHR) models exhibited hypotensive effects, but to normal rats no change in blood pressure was observed. Further, the hypotensive effects of the peptide were observed for a long time unlike effects generated by renin inhibitor (H6137 from Sigma) or renin neutralizing antibody. Therefore, it may be concluded that the hypotensive effect obtained by the peptide is not ascribed to the inhibited renin activity (International Publication WO01/077673). Further, the partial peptide derived from a rat prorenin profragment significantly decreased Ang II in the kidney of STZ rats but brought about no change in Ang II in normal rats (International Publication W02004/073740). It is predicted that these effects were caused by the inhibition of in vivo nonenzymatic activation of rat prorenin by the peptide described above.
Here, the activity of the nonenzymatically activated prorenin is similar to that of enzymatic effect of mature renin (referred to as "renin-like activity") but different from that of the mature renin.

A peptide contained in the tumor-growth suppressor of the present invention as an active ingredient is believed to inhibit tumor angiogenesis and exhibit tumor-growth suppressive effects by antagonizing binding of the prorenin and the binding protein thereof and exerting suppressive effects on nonenzymatic activation of prorenin.
Further, it is believed that the peptide described above has no inhibiting effect on the renin activity due to its mechanism of action, and that is an important feature of the tumor-growth suppressor of the present invention. Unlike renin inhibitor, this feature can inhibit the prorenin activation in a clinical state without inhibiting RAS, which plays a key role in life-support system in living organisms, and can suppress the antagonistical inhibition-induced production of tissue Ang II in the downstream region. The tumor-growth suppressor of the present invention does not exhibit direct suppressive effect on tumor cell proliferation as widely known anticancer drugs.

The amino acid sequence of prorenin profragment is known (SEQ. ID. NO: 1 below).
(SEQ. ID. NO: 1)
LPTDTTTFKR IFLKRMPSIR ESLKERGVDM ARLGPEWSQP MKR
The partial peptide of the present invention, which is an active ingredient, consists of an amino acid sequence selected within a sequence from the 1st leucine to the 18th serine in the N-terminus (LPTDTTTFKR IFLKRMPS (SEQ. ID. NO : 2)). Such a partial peptide has a sequence of 2 to 8, preferably 2 to 5 continuous amino acids. More preferably, the partial peptide has an amino acid sequence comprising at least any one of the sequences from the 11th to the 13th, from the 13th to the 15th and from the 11th to the 15th in the N-terminus. For such a partial peptide, a peptide consisting of an amino acid sequence consisting of a sequence from the 11th to the 18th in the N-terminus is preferably used. The active ingredient of the present invention may be a peptide equivalent to the partial peptide described above. Amino acids composing such equivalent peptide are not necessarily identical to the amino acid sequence in the profragment site. The equivalent peptides may be those having same effects as the partial peptide described above has, namely those having inhibiting effects on nonenzymatic activation of prorenin, and includes peptides made by introducing, as appropriate, mutations such as deletion, substitution, addition and insertion into the amino acid sequence of the partial peptide described above. Of course, the tumor-growth suppressor of the present invention may be the one comprising one kind of the peptide described above or the one comprising the combination of the two kinds or more.

These peptides can be produced by various methods widely known in the art. For example, they can be synthesized by solid-phase method.

To formulate the tumor-growth suppressor of the present invention, well known methods can be applied as appropriate depending on the physical properties of the peptide of interest. For example, methods for formulating tablets, capsules, water- and ethanol-based agent, liposome agent, fat emulsion, inclusion complex such as cyclodextrin can be used.
Dispersants, pills, capsules and tablets can be produced using excipient such as lactose, disintegrant such as starch, lubricant such as magnesium stearate, binder such as polyvinyl alcohol, surfactant such as fatty acid ester and plasticizer such as glycerin. To produce tablets and capsules, pharmaceutical solid supports are used.
Suspensions can be produced using water, saccharide such as sucrose, glycol such as PEG, and oil.
Injection solution can be prepared using carriers comprising salt solution, glucose solution or a mixture of brine and glucose solution.
Liposome-encapsulation can be performed by, for example, adding a solution containing the substance of interest dissolved in a solvent (such as ethanol) to a solution containing phospholipid dissolved in an organic solvent, distilling the solvent off, adding phosphate buffer there, shaking, followed by sonication, centrifugation and filtration to collect the supernatant.
Fat emulsification may be performed by, for example, mixing the substance of interest , oil component (such as vegetable oil), emulsifier (such as phospholipid), heating the mixture into a solution and conducting emulsification and homogenization using an emulsifying device after adding a necessary amount of water for that. Further, the resultant can be freeze-dried. Meanwhile, when conducting fat-emulsification, emulsifying aid which includes glycerin, sugar and the like may be added.
Cyclodextrin clathration may be performed by, for example, adding a solution containing cyclodextrin in water heated for dissolution to a solution containing the substance of interest dissolved in a solvent (such as ethanol), cooling to precipitate and filtering followed by sterilization dry. In that case, cyclodextrin used may be selected depending on the size of the substance of interest as appropriate from those having variety of cavity diameters (type α, β or γ).

The tumor-growth suppressor of the present invention can be administered parenterally in the form of, for example, injectables for intravenous and intramuscular injections and the like and suppositories, and administered transdermally, intraperitoneally or orally. In view of the stability and absorption, parenteral administration is preferred. The dosage will be determined as appropriate depending on the individual case and considering the symptom, age, sex and the like of the subject to be administered, and usually the dosage in a range between 0.05 and 1000 mg/kg, preferably between 0. 1 and 50 mg/kg and more preferably between 1 and 20 mg/kg per adult per day is administered. For instance, intravenous administration from once to several times a day, continuous intravenous administration for 1 to 24 hours a day or subcutaneous administration may be continuously performed. Of course, as mentioned above, the dosage will be different depending on various conditions, so that the amount less than the dosage range described above may be sufficient. The injectables in terms of its stability can be filled in a vial and the like, then frozen to remove water by usual freeze-drying, and administered after repreparing the solution from the freeze-dried substances just before use.

The application of the tumor-growth suppressor of the present invention is not limited to specific types of tumor, but it is preferably used for a solid tumor. As a solid tumor, for example, liver cancer can be mentioned.

### (Example)

The present invention will further be explained in detail below with reference to examples, but the present invention will not be limited by them and many modifications can be made without departing from the technical concept of the present invention.

### Example 1

A peptide having IFLKRMPS (SEQ. ID. NO: 3), which is a sequence from the 11th isoleucine to the 18th serine in the N-terminus derived from human prorenin, was synthesized in the usual manner by solid-phase method. In the following, h-human prorenin peptide (also referred to as "pf11-18 peptide") was used as the present peptide.

### Experimental Example 1

### Method

The following experiment was conducted using nude mice transplanted with human hepatocellular carcinoma.
25 five-week-old BALB/c nu/nu mice were prepared. Tumor grown from implanted human hepatocellular carcinoma (HCC) was cut into about 50 small pieces and implanted into 23 of 25 five-week-old (male) BALB/c nu/nu mice. HCC cell pieces were spread over a dish into which 5 mL of germ-free saline was added, and implanted, one at a time, into the nude mice in the right-hand side of the left and right back. The incision made by scissors was closed just by tweaking with tweezers and disinfected with 70% ethanol-soaked cotton swab. Then, the incision was left untouched until naturally closed. Usually, the incision was well closed in a day or so.
Then, the h-prorenin peptide of the present invention (pf11-18 peptide) was intraperitoneally administered to 5 mice at 10 mg/kg/day and 1 mg/kg/day respectively everyday. The administered peptide solutions were prepared such that the concentrations were respectively 0.25 mg/mL and 2.5 mg/mL in sterilized saline. 5 mg/vial pf11-18 peptide was prepared by dissolving pf11-18 peptide in sterilized saline at 5 mg/mL and diluting it 2-fold with sterilized saline and stored at -20°C. 5 mg/vial pf11-18 peptide was prepared by dissolving pf11-18 peptide in sterilized saline at 5mg/mL and adding 0. 5 mL of it to 9.5 mL of sterilized saline to dilute 20-fold and then stored at -20°C after placing it in a vial at 1 mL each. Before the administration, their body weights were measured and approximately 100 to 150 µL of them were injected using a 25G injection needle and a tuberculin syringe. Sterilized saline was used for negative control and captopril (ACE inhibitor) for positive control in such a manner that the concentration of captopril was 2.5 mg/mL in sterilized saline to administer at 10 mg/kg/day.

The conditions of mice were recorded with digital camera and video and body weights and tumor diameters were measured everyday. The measurement of the tumor diameter was conducted by measuring the length and the width of it using a caliper from the 13th day after the implantation of the liver cancer.
During the experiment, the hepatocellular carcinoma implanted in mice grew steadily, and on the 22nd day after the implantation of the liver cancer, necrosis was observed in a part of tumors, so that tumors were extirpated. On the 22nd day, the final body and tumor weights were measured, and the liver cancer tumor and the serum were sampled. The final weight measurement was conducted with the collected tumors. The sampling of the liver cancer tumor was conducted as follows. At first, the half of the extirpated tumor was soaked in 10% formalin fixative to store in masses. The remained half of the extirpated tumor was divided into a few pieces and stored at -80°C. The serum sampling was conducted by respectively collecting the blood from the heart of each mouse and subjecting it to serum separation. Approximately 200 µL of serum was collected from 500 µL of blood and stored at -20°C. The amount of endogenous prorenin in the serum was measured.

### Result

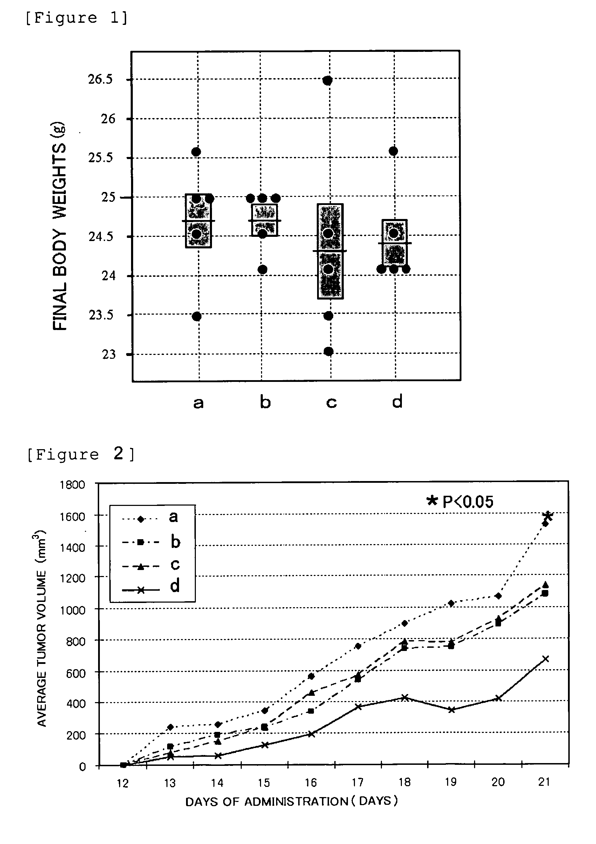

Results were shown in Tables 1 and 2 and Figs. 1 and 2 (note: no mouse died with the dosage in the present experimental example and no visual side-effect was observed.)
Table 1 shows the measurement results of the amount of endogenous prorenin.

**[Table 1]**

| | | Prorenin (pg/ml) |
|---|---|---|
| Saline administered to the | 1 | 293 |
| human liver cancer implanted | 2 | 36.6 |
| nude mouse serum | 3 | N.D |
| | 4 | N.D |
| | 5 | N.D |
| Human liver cancer not | 1 | N.D |
| implanted nude mouse serum | 2 | N.D |

Fig. 1 shows the comparison of the final body weights on the 22nd day. Little difference was observed in the final body weights in all groups.
Table 2 and Fig. 2 show chronological changes in the average tumor volumes of human liver cancer-implanted nude mice from the 12th day to the 21st day. The suppression of tumor volume increase was observed in the (c) pf11-18 peptide-administered group at 1 mg/kg/day, the (d) pf11-18 peptide-administered group at 10 mg/kg/day and the (b) captopril-administered group in comparison with the (a) saline-administered group of the negative control. The (d) pf11-18 peptide-administered group at 10 mg/kg/day exhibited a higher suppressive effect on tumor volume increase than the (c) pf11-18 peptide-administered group at 1 mg/kg/day. Further, the (d) pf11-18 peptide-administered group at 10 mg/kg/day had a higher suppressive effect on tumor volume increase than the (b) captopril-administered group at 10 mg/kg/day.

**[Table 2]**

| Chronological changes in the average tumor volumes of human liver cancer-implanted nude mice | | | | | | | | | | |
|---|---|---|---|---|---|---|---|---|---|---|
| Days of administration | 12 | 13 | 14 | 15 | 16 | 17 | 18 | 19 | 20 | 21 |
| Saline | 0.0 | 241.3 | 259.8 | 342.3 | 565.1 | 751.6 | 897.1 | 1022.5 | 1068.2 | 1528.0 |
| Captopril 10mg/kg | 0.0 | 117.6 | 189.3 | 241.8 | 340.7 | 541.3 | 737.2 | 748.8 | 890.3 | 1080.1 |
| pf11- 18 peptide 1mg/kg | 0.0 | 74.1 | 147.3 | 243.6 | 461.1 | 567.1 | 783.5 | 778.8 | 924.6 | 1141.2 |
| pf11-18 peptide 10mg/kg | 0.0 | 54.3 | 54.7 | 125.9 | 196.8 | 368.6 | 422.8 | 342.8 | 420.4 | 669.0 |

### (Industrial Applicability)

As explained above, the novel tumor-growth suppressor of the present invention can provide more excellent tumor-growth suppressive effects than conventionally known angiotensin-converting enzyme inhibitors and the like. It is believed that the present tumor-growth suppressor used for treating tumor will have fewer side effects and is more likely to be effective for sick patients showing no effects through the use of tumor-growth suppressors such as ACE inhibitor.

## Claims

1. A tumor-growth suppressor comprising as an active ingredient a polypeptide consisting of a partial sequence selected within a sequence from the 1st to the 18th in the N-terminus of an amino acid sequence in the prorenin profragment region or equivalent peptide thereof.

2. The tumor-growth suppressor according to claim 1, wherein said partial sequence is a sequence of 2 to 8 amino acids.

3. The tumor-growth suppressor according to claim 1, wherein said partial sequence is an amino acid sequence comprising at least any one of the sequences from the 11th to the 13th, from the 13th to the 15th and from the 11th to the 15th in the N-terminus.

4. The tumor-growth suppressor according to claim 1, wherein said partial sequence is an amino acid sequence from the 11th to the 18th in the N-terminus.

5. The tumor-growth suppressor according to any one of claims 1 to 4, wherein the tumor-growth suppressor is used for a solid tumor.

6. The tumor-growth suppressor according to claim 5, wherein the tumor-growth suppressor is used for liver cancer.


SEQUENCE LISTING

<110>  Protein Express Co., Ltd.

<120>  A tumor growth inhibitor

<130>  GP06-1004PCT

<150>  JP2005-165295
<151>  2005-06-06

<160>  3

<170>  PatentIn version 3.1

<210>  1
<211>  43
<212>  PRT
<213>  Homo sapiens

<400>  1

Leu Pro Thr Asp Thr Thr Thr Phe Lys Arg Ile Phe Leu Lys Arg Met
1               5                   10                  15

Pro Ser Ile Arg Glu Ser Leu Lys Glu Arg Gly Val Asp Met Ala Arg
            20                  25                  30

Leu Gly Pro Glu Trp Ser Gln Pro Met Lys Arg
        35                  40

<210>  2
<211>  18
<212>  PRT
<213>  Homo sapiens

<400>  2

Leu Pro Thr Asp Thr Thr Thr Phe Lys Arg Ile Phe Leu Lys Arg Met
1               5                   10                  15

Pro Ser

<210>  3
<211>  8
<212>  PRT
<213>  Homo sapiens

<400>  3

Ile Phe Leu Lys Arg Met Pro Ser
1               5